Europäisches Patentamt

European Patent Office

Office européen des brevets

(19)

(11) Veröffentlichungsnummer : **0 363 310 B1**

# (12) EUROPÄISCHE PATENTSCHRIFT

(45) Veröffentlichungstag der Patentschrift :
27.11.91 Patentblatt 91/48

(51) Int. Cl.⁵ : **A61F 2/06**

(21) Anmeldenummer : **89810626.5**

(22) Anmeldetag : 23.08.89

(54) **Verfahren zum Beschichten von schlauchförmigen Prothesen, insbesondere Gefässprothesen.**

(30) Priorität : 07.10.88 CH 3751/88

(43) Veröffentlichungstag der Anmeldung :
11.04.90 Patentblatt 90/15

(45) Bekanntmachung des Hinweises auf die
Patenterteilung :
27.11.91 Patentblatt 91/48

(84) Benannte Vertragsstaaten :
AT DE FR GB IT

(56) Entgegenhaltungen :
WO-A-82/03764
DE-A- 3 637 260

(73) Patentinhaber : **GEBRÜDER SULZER
AKTIENGESELLSCHAFT**
**Zürcherstrasse 9**
**CH-8401 Winterthur (CH)**

(72) Erfinder : **Müller, Werner, Dr.**
**Wannenstrasse 6**
**CH-8542 Wiesendangen (CH)**
Erfinder : **Hensel, Marie-Claude**
**Breitistrasse 62**
**CH-8614 Bertschikon (CH)**
Erfinder : **Huber, August**
**Gotthelfstrasse 11**
**CH-8352 Räterschen (CH)**

## Beschreibung

Die Erfindung betrifft ein Verfahren zum Beschichten von schlauchförmigen Prothesen aus Kunststoff, z.B. Gefässprothesen mit lebenden Zellen, die als wässrige Suspension in physiologischer Lösung in dem Gefäss ausgesät und infolge der Schwerkraft darauf abgelagert werden, wobei die mit der Suspension gefüllte, in horizontaler Richtung ausgespannte Prothese, die zuvor mit einer extrazellulären Matrix beschichtet worden ist und in von Absetzpausen unterbrochenen Schritten um eine Längsachse gedreht wird. Ein solches Verfahren ist aus der WO 82/03764 bekannt.

Für das Beschichten von schlauchförmigen Prothesen mit lebenden Zellen, insbesondere mit Monoschichten epithelialer Zellen, ist das vorstehend geschilderte Verfahren üblich. In der Praxis hat sich gezeigt, dass auch bei "weiträumigen" Variationen der Schrittwinkel für die Drehung und/oder der Dauer der Absetzpausen ein gleichmässiger Belag von Zellen auf der Innenwand der Prothese nicht erreicht wird, sondern sich Streifenmuster bilden, und die Beschichtungen keine reproduzierbaren Ergebnisse bringen.

Aufgabe der Erfindung ist es, dass immer eine gleichmässige Verteilung der Zellen in Umfangsrichtung und eine lückenlose (konfluente) Beschichtung der Protheseninnenwand erreicht wird ; um einen guten "Wirkungsgrad" zu erreichen, sollen dabei möglichst alle lebenden und lebensfähigen Zellen aus der Suspension abgeschieden werden.

Diese Aufgabe wird mit der Erfindung durch die Anwendung folgender Merkmale gelöst :
— Beginn Aussaat bei einer tiefen Temperatur von < 5°C ;
— Erwärmung auf etwa Körpertemperatur unter kontinuierlichem Drehen zur Homogenisierung der Suspension und Verhinderung von verfrühtem, unkontrolliertem Absetzen ;
— Kurze erste Absetzpause von maximal 2 sec ;
— Drehung um einen Winkel, der — neben gegebenenfalls von ganzzahligen Vielfachen von $2\pi$
— folgende Kriterien erfüllt :
    a) Nahe $\pi$, jedoch ungleich $\pi$ ;
    b) restloser Bruchteil von ohne gemeinsamen Teiler ;
    c) $4/5\,\pi \leq$   Winkel $\leq$   $8/9\,\pi$, oder
    d) $10/9\,\pi \leq$   Winkel $\leq$   $6/5\,\pi$ ;
— Bis zu einer integrierten Gesamtzeit von einigen Stunden :
Unter schrittweiser Erhöhung der Pausendauer um einen geringen Betrag von 1 bis 2 sec alternierend mit Drehungen um den gewählten Winkel ;
— Kontinuierliches Drehen bis zum Absaugen der Restsuspension mit den nicht abgeschiedenen Zellen.

Die tiefe Temperatur zu Beginn der Aussaat — d.h. bei einem Füllen der Prothese, die beispielsweise aus einem der in der Gefässprothetik verwendeten Kunststoffe besteht, mit einer zellhaltigen, wässrigen Suspension in physiologischer Lösung — gewährleistet, dass eine Belegung der Prothese mit Zellen in der Vorbereitungsphase für die eigentlichen Beschichtungsschritte nicht erfolgt. Nach Erreichen von etwa Körpertemperatur z.B. 37°C, ausgehend von einer ersten kurzen Absetzpause, innerhalb der sich sicher ebenfalls noch keine Zellen absetzen, werden die Pausen zwischen den einzelnen Drehungen sukzessive, schrittweise gesteigert, wobei nicht unbedingt bei jedem Schritt eine Pausenverlängerung erfolgen muss. Die Pausenverlängerungen haben jeweils geringe Beträge zwischen 1 bis 2 sec, die konstant sein können, aber nicht müssen. Durch diese schrittweisen Pausenverlängerungen erhalten alle Zellen, deren Absetzzeiten bzw. -geschwindigkeiten sowohl innerhalb einer Zellinie als auch von verschiedenen Zellinien ein ganzes Spektrum bilden, das auch vom Wachstumsstadium der Zellen abhängig sein kann, die Möglichkeit sich abzusetzen.

Die Kriterien für die Winkel, um die die Drehungen zwischen den Pausen erfolgen, stellen sicher, dass jedes Wandelement in Umfangsrichtung im Mittel einer gleichen Dauer an Absetzwirkung unterworfen ist, wobei die angegebenen Grenzen verhindern, dass es zu Streifenbildungen entweder durch Ueberlappungen der oder durch Leerräume zwischen den — in den einzelnen Pausen einem Absetzen der Zellen unterworfenen — Winkelbereichen in Umfangsrichtung kommt. Die Drehwinkel können um ganzzahlige Vielfache von $2\pi$ erweitert werden, ohne dass die Wirkung des neuen Verfahrens beeinträchtigt wird ; gegebenenfalls können derartige Erweiterungen sogar vorteilhaft sein, da dadurch eine Durchmischung der Zellen und damit eine Zwischenhomogenisierung der Suspension bewirkt wird.

Drehwinkel nahe $\pi$ bewirken darüberhinaus, dass in allen Bereichen Zellen jeder Absetzgeschwindigkeit, also sowohl schnell als auch langsam sich absetzende Zellen, auf den ganzen Umfang verteilt nacheinander abgelagert werden. Als besonders vorteilhaft haben sich dabei im Experiment Drehwinkel von $4/5\,\pi$ oder $6/5\,\pi$ erwiesen bei denen der während einer Pause auf dem Umfang erfasste Absetzbereich $\pi/5$ beträgt.

Haben die Pausen schliesslich Werte von einigen, beispielsweise zwischen 2 und 3 Minuten erreicht, so sind in der Suspension nur noch sich sehr langsam absetzende, geschädigte und tote Zellen vorhanden, die entfernt werden müssen. Ein Absetzen der noch in der Suspension vorhandenen Zellen bis zum Absaugen dieser Restsuspension wird verhindert durch kontinuierliches Weiterdrehen der Prothese.

Mit Vorteil dient das neue Verfahren dazu, eine Prothese mit einer Monoschicht epithelialer Zellen, z.B. von Endothel-Zellen oder einer ein- oder mehrlagigen Schicht aus einem oder mehreren Zelltypen, zu belegen. Bei der Verwendung einer Anzahl von Kunststoffen, beispielsweise von mit Poren versehenem Polyurethan, ist es zweckmässig, die Prothese vor jeglicher Beschichtung einer Aequilibrier-Behandlung zu unterziehen, bei der die äussere und die innere Oberfläche des Kunststoff-Schlauches sowie der Kunststoff selbst mit der Umgebungsflüssigkeit ins Gleichgewicht gebracht werden, d.h. beispielsweise wassergesättigt werden, um beispielsweise die Poren zu öffnen und durchlässig zu machen.

Im folgenden wird die Erfindung anhand eines Ausführungsbeispiels näher erläutert.

Es bestehe die Aufgabe eine Gefässprothese aus einem Kunststoff-Schlauch, deren Herstellung bereits in der Parallelanmeldung EP-A-0323397 beschrieben worden ist mit einer einlagigen Schicht Endothel-Zellen zu beschichten.

Die Beschichtung erfolgt in einer in der Parallelanmeldung EP-A-0320441 bereits beschriebenen Behandlungskammer in der die Prothese in Längsrichtung ausgespannt und um eine Längsachse, die nicht notwendigerweise konzentrisch mit der Prothesenachse ist, drehbar ist. Als Rotationsantrieb dient in bekannter Weise z.B. ein konventioneller Schrittmotor, an dessen Welle die Behandlungskammer angeschlossen ist.

Wie erwähnt, ist es für eine Prothese, wie sie dem Ausführungsbeispiel zugrundeliegt, zweckmässig eine Aequilibrierungs-Behandlung durchzuführen, um die Prothesenwand mit Wasser zu sättigen und alle Poren zu öffnen. Für diese Vorbehandlung werden die völlig unbeschichteten Prothesen zuerst in bidestilliertem Wasser autoklaviert und dann in der Behandlungskammer Aussen- und Innenraum im Bereich der Prothese mit einer bekannten Pufferlösung HBS-2 gefüllt. HBS-2 ist eine physiologisch gepufferte Salz-Lösung, die unter dem Namen "Hepes buffered saline" bekannt ist, aber zusätzlich mit Kalzium- und Magnesiumchlorid modifiziert wird. Diese Behandlung erfolgt ohne besondere thermostatische Vorkehrungen bei Umgebungs-Temperatur; sie besteht einfach darin, dass die mit der Lösung gefüllte Kunststoff-Prothese einige Tage, beispielsweise drei Tage, stehenbleibt, eine Zeit, die sich nach der Art des Kunststoffes und nach der Wandstruktur der Prothese richtet.

Anschliessend wird die äquilibrierte Prothese in bekannter Weise auf ihrer Innenseite mit einer Vorbeschichtung aus einer extrazellulären Matrix versehen; eine solche besteht bekanntlich aus Fibronektin, Kollagen, Laminin, Elastin oder Thrombospondin bzw. natürlichen oder definierten Gemischen einzelner dieser und weiterer Stoffe. Im vorliegenden Beispiel wird für die Vorbeschichtung Fibronektin verwendet. Die Beschichtung erfolgt nach einem für die Beschichtung von Kulturgefässen üblichen Verfahren. Im einzelnen wird zunächst die Pufferlösung der Aequilibrier-Behandlung aus Behandlungskammer und Prothese entfernt und diese mit einer Fibronektin-Lösung gefüllt; anschliessend wird in den die Prothese umgebenden Aussenraum der Kammer wiederum Pufferlösung eingebracht, um ein Austrocknen der Prothesenwand von aussen her zu verhindern.

Nach einer üblichen Einwirkungszeit der Fibronektin-Lösung auf die Protheseninnenwand von beispielsweise etwa 45 min bei Umgebungstemperatur wird die gefüllte Behandlungskammer in einem Kühlschrank gelagert oder in Eiswasser gestellt bis eine Abkühlung auf etwa 4°C erreicht ist.

Als nächster Schritt erfolgt nunmehr die Aussaat der Endothel-Zellen in die mit Fibronektin beschichtete Prothese. Aus der gekühlten Behandlungskammer werden Pufferlösung und Fibronektin-Lösung entfernt und der Protheseninnenraum mit, ebenfalls auf 4°C abgekühlter, Zellsuspension gefüllt. Bei der tiefen Temperatur zeigen die Zellen in der Suspension keine Tendenz zum Absetzen. Der Zellenbedarf beträgt in bekannter Weise etwa $10^5$ Zellen/cm² zu beschichtender Prothesenoberfläche. In den Aussenraum der Prothese wird eine übliche Nährlösung hinzugefügt, bei der es sich beispielsweise ebenfalls um eine im Handel erhältliche physiologische Nähr- und Kultur-Lösung handelt.

Nach dem Aufsetzen der in der geschilderten Weise behandelten Prothese bzw. der noch gekühlten Behandlungskammer auf den erwähnten Schritt- oder Positionierungsmotor erfolgt die eigentliche Beschichtung der Protheseninnenwand mit den Zellen. Der erste Teilschritt dafür besteht in einem Erwärmen der ganzen Behandlungskammer auf etwa Körpertemperatur, d.h. ungefähr 37°C, unter kontinuierlichem Drehen der Kammer mit 20 bis 50 U/min., insbesondere beispielsweise mit 33 U/min.; durch die kontinuierliche Drehung werden Sedimentationen innerhalb der Zellsuspension verhindert.

Ist die erforderliche Körpertemperatur erreicht, so beginnt das Sedimentieren der Zellen mit einer Pause von 2 sec, nach der eine Drehung um 6/5 π erfolgt; nach der Drehung folgt wieder eine Absetzpause, deren Dauer um etwa 1 bis 2 sec. verlängert ist, ehe eine weitere Drehung um den genannten Winkel anschliesst. Alternierend mit Pausenverlängerungen um jeweils 1 bis 2 sec — die bei jedem Schritt oder auch erst nach 2 oder 3 Schritten vorgenommen werden — wird die Prothese immer um den gleichen Winkel fortschreitend gedreht, bis Pausenzeiten von etwa 2 bis 3 min. erreicht sind. Daraus ergibt sich eine gesamte Beschichtungsdauer von einigen Stunden, während der die ganze Prothese innen gleichmässig mit Endothel-Zellen belegt wird, die nach ihrer Ausbreitung die Prothese lückenlos und gleichmässig bedecken. Auf den ganzen Umfang sind dabei schnell und langsam sich absetzende Zellen weitgehend gleichmässig verteilt, so dass eine gleichmässige Beschichtung auf der ganzen Fläche vorhanden ist.

Nach dem Ende des eigentlichen Beschichtens sind in der Suspension, wie bereits erwähnt, nur noch sich sehr langsam absetzende, wenig lebensfähige und tote Restzellen vorhanden, die abgesaugt werden müssen. Die Behandlungskammer wird daher nach dem Beschichten mit einer Drehzahl von 1 bis 5 U/min. kontinuierlich weitergedreht, um ein Absetzen dieser Restzellen auf der beschichteten Prothese zu verhindern.

Während dieser Nachbehandlung oder unmittelbar nach dem Abstellen der Drehvorrichtung wird dann die nun zellarme Suspension mit den Restzellen abgesaugt.

Die beschichtete Prothese wird nun in der gleichen Behandlungskammer einer Konditionierungsbehandlung unterworfen, wie sie beispielsweise in der Parallelanmeldung EP-A-0320441 beschrieben ist.

**Patentansprüche**

1. Verfahren zum Beschichten von schlauchförmigen Gefässprothesen aus Kunststoff mit lebenden Zellen, die als wässrige Suspension in physiologischer Lösung in dem Gefäss ausgesät und infolge der Schwerkraft darauf abgelagert werden, wobei die mit der Suspension gefüllte, in horizontaler Richtung ausgespannte Prothese, die zuvor mit einer extrazellulären Matrix beschichtet worden ist, in von Absetzpausen unterbrochenen Schritten um eine Längsachse gedreht wird, gekennzeichnet durch die folgenden Merkmale :
— Beginn der Aussaat bei einer tiefen Temperatur von < 5°C ;
— Erwärmung auf etwa Körpertemperatur unter kontinuierlichem Drehen zur Homogenisierung der Suspension und Verhindern von verfrühtem, unkontrolliertem Absetzen der Zellen ;
— Kurze erste Absetzpause von maximal 2 sec ;
— Drehung um einen Winkel, der — neben gegebenenfalls ganzzahligen Vielfachen von 2 π — folgende Kriterien erfüllt :
a) Nahe π, jedoch ungleich π,
b) restloser Bruchteil von ohne gemeinsamen Teiler,
c) 4/5 π ≤ Winkel ≤ 8/9 π, oder
d) 10/9 π ≤ Winkel ≤ 6/5 π,
— Bis zu einer integrierten Gesamtzeit von einigen Stunden :
Unter schrittweiser Erhöhung der Pausendauer um einen geringen Betrag von 1 bis 2 sec alternierend mit Drehungen um den gewählten Winkel ;
— Kontinuierliches Drehen bis zum Absaugen der Suspension mit den nicht abgeschiedenen Zellen.

2. Verfahren nach Anspruch 1, dadurch gekennzeichnet, dass die Prothese, bevor irgendeine Beschichtung vorgenommen wird, einer Äquilibrier-Behandlung unterworfen wird, bei der eine Sättigung des Kunststoff-Schlauches mit wässriger physiologischer Salz- oder Nährlösung erfolgt.

3. Verfahren nach Anspruch 1 oder 2, dadurch gekennzeichnet, dass die Gefässprothese mit einer Monoschicht epithelialer Zellen belegt wird.

4. Verfahren nach Anspruch 1 oder 3, dadurch gekennzeichnet, dass die Gefässprothese mit einer ein- oder mehrlagigen Schicht aus einem oder mehreren Zelltypen belegt wird.

5. Verfahren nach einem der Ansprüche 1 bis 4, dadurch gekennzeichnet, dass der Drehwinkel 4/5 π oder 6/5 π beträgt.

6. Verfahren nach einem der Ansprüche 1 bis 5, dadurch gekennzeichnet, dass die Drehwinkel zwischen den Absetzpausen um ganzzahlige Vielfache von 2 π erweitert werden.

**Revendications**

1. Procédé pour recouvrir des prothèses vasculaires tubulaires en matière plastique avec des cellules vivantes dont on ensemence le vaisseau, sous la forme d'une suspension aqueuse dans une solution physiologique et qui s'y déposent, sous l'effet de la gravité, la prothèse remplie de la suspension, tendue en direction horizontale et qui a été recouverte auparavant avec une matrice extracellulaire, étant soumise à un rotation autour d'un axe longitudinal, en étapes interrompues par des pauses de dépôt, caractérisé par les particularités suivantes :
— début de l'ensemencement à une basse température < 5°C ;
— échauffement à la température corporelle environ sous une rotation continue, afin d'homogénéiser la suspension et d'empêcher le dépôt prématuré, non contrôlé, des cellules ;
— première courte pause de dépôt de 2 s au maximum ;
— rotation d'un angle qui — outre éventuellement des multiples entiers de 2 π — remplit les critères suivants :
a) voisin de π, mais différent de π,
b) fraction sans reste de π sans diviseur commun,
c) 4/5 π ≤ angle ≤ 8/9 π, ou
d) 10/9 π ≤ angle ≤ 6/5 π,
— jusqu'à un temps total intégré de quelques heures : avec augmentation progressive de la durée des pauses, d'une petite durée de 1 à 2 s en alternance avec des rotations de l'angle choisi ;
— rotation continue jusqu'à élimination par succion de la suspension avec les cellules non déposées.

2. Procédé selon la revendication 1, caractérisé en ce qu'avant de procéder à tout recouvrement, la prothèse est soumise à un traitement d'équilibrage pour lequel il se produit une saturation du tuyau en matière plastique avec une solution physiologique aqueuse saline ou nutritive.

3. Procédé selon la revendication 1 ou 2, caractérisé en ce que la prothèse vasculaire est recouverte d'une monocouche de cellules épithéliales.

4. Procédé selon la revendication 1 ou 3, caractérisé en ce que la prothèse vasculaire est recouverte avec une couche simple ou multiple faite d'un ou de plusieurs types de cellules.

5. Procédé selon l'une des revendications 1 à 4, caractérisé en ce que l'angle de rotation est égal à 4/5 π ou 6/5 π.

6. Procédé selon l'une des revendications 1 à 5, caractérisé en ce que les angles de rotation sont augmentés de multiples entiers de 2 π, entre les pauses de dépôt.

## Claims

1. A process for coating tubular vessel prostheses made from plastics with living cells which are sown as an aqueous suspension in physiological solution in the vessel and are deposited thereon by gravity, the prosthesis filled with suspension and stretched in the horizontal direction having previously been coated with an extra-cellular matrix and being rotated about a longitudinal axis in steps interrupted by deposition pauses, characterised by the following features :
— Start of sowing at a low temperature of < 5°C;
— Heating to approximately body temperature with continuous rotation to homogenise the suspension and prevent premature uncontrolled deposition of the cells ;
— Short first deposition pause of two seconds maximum ;
— Rotation about an angle which — in addition to possibly integral multiples of 2 π — satisfies the following criteria :
  a) Near π, but not equal to π,
  b) Complete fraction of π without common divisor,
  c) $4/5 \pi \leq$ angle $\leq 8/9 \pi$, or
  d) $10/9 \pi \leq$ angle $\leq 6/5 \pi$,
— Up to an integrated total time of some hours : With stepwise increase in pause duration by a small amount of 1 to 2 seconds alternating with rotations about the selected angle ;
— Continuous rotation until the suspension with the non-separated cells has been removed by suction.

2. A process according to claim 1, characterised in that before any coating is carried out the prosthesis is subjected to an equilibrating treatment, in which the plastic tube is saturated with aqueous physiological salt or nutrient solution.

3. A process according to claim 1 or 2, characterised in that the vessel prosthesis is coated with a mono-coating of epithelial cells.

4. A process according to claim 1 or 3, characterised in that the vessel prosthesis is coated with a single-layer or multi-layer coating of one or more cell types.

5. A process according to any one of claims 1 to 4, characterised in that the angle of rotation is 4/5 π or 6/5 π.

6. A process according to any one of claims 1 to 5, characterised in that the angles of rotation between the deposition pauses are increased by integral multiples of 2 π.